(19) Europäisches Patentamt
European Patent Office
Office européen des brevets

(11) Veröffentlichungsnummer: **0 135 083**
**A2**

(12) # EUROPÄISCHE PATENTANMELDUNG

(21) Anmeldenummer: **84109025.1**

(22) Anmeldetag: **31.07.84**

(51) Int. Cl.[4]: **C 07 D 405/06**
A 01 N 43/653

(30) Priorität: **11.08.83 DE 3329128**

(43) Veröffentlichungstag der Anmeldung:
**27.03.85 Patentblatt 85/13**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Reiser, Wolf, Dr.**
**Kiebitzweg 12a**
**D-5600 Wuppertal 1(DE)**

(72) Erfinder: **Krämer, Wolfgang, Dr.**
**Am Eckbusch 39/45**
**D-5600 Wuppertal 1(DE)**

(72) Erfinder: **Büchel, Karl Heinz, Prof. Dr.**
**Dabringhausener Strasse 42**
**D-5093 Burscheid(DE)**

(72) Erfinder: **Brandes, Wilhelm, Dr.**
**Eichendorffstrasse 3**
**D-5653 Leichlingen(DE)**

(72) Erfinder: **Reinecke, Paul, Dr.**
**Lessingstrasse 11**
**D-5090 Leverkusen 3(DE)**

(54) **3-Cycloalkyl-1-(1,3-dioxan-5-yl)-2-(1,2,4-triazol-1-yl)-propan-1-one und -propan-1-ole.**

(57) Die Erfindung betrifft neue 3-Cycloalkyl-1-(1,3-dioxan-5-yl)-2-(1,2,4-triazol-1-yl)-propan-1-one und -propan-1-ole, mehrere Verfahren zu ihrer Herstellung und ihre Verwendung als Pflanzenschutzmittel.

Die Verbindungen der allgemeinen Formel

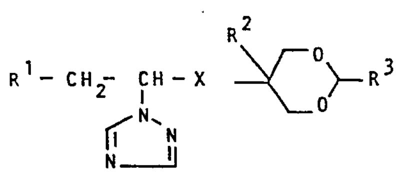

(I)

in welcher

$R^1$, $R^2$, $R^3$ und X die in der Beschreibung angegebene Bedeutung besitzen,

werden beispielsweise erhalten, wenn man 3-Cycloalkyl-1-(1,3-dioxyn-5-yl)-2-halogen-ketone mit Triazol in Gegenwart eines Verdünnungsmittels und eines Säurebindemittels umsetzt.

Die Wirkstoffe sind für den Gebrauch als Pflanzenschutzmittel geeignet und können mit besonders gutem Erfolg zur Bekämpfung von Getreidekrankheiten, von Obstkrankheiten oder von Gemüsekrankheiten eingesetzt werden.

Daneben besitzen die erfindungsgemäßen Wirkstoffe auch eine gute Wirksamkeit gegen die Erreger von Reiskrankheiten.

EP 0 135 083 A2

BAYER AKTIENGESELLSCHAFT 5090 Leverkusen, Bayerwerk

Konzernverwaltung RP
Patentabteilung Slr/Kü 10. Aug. 1983
Ib

3-Cycloalkyl-1-(1,3-dioxan-5-yl)-2-(1,2,4-triazol-1-yl)-propan-1-one und -propan-1-ole

Die Erfindung betrifft neue 3-Cycloalkyl-1-(1,3-dioxan-5-yl)-2-(1,2,4-triazol-1-yl)-propan-1-one und -propan-1-ole, mehrere Verfahren zu ihrer Herstellung und ihre Verwendung als Pflanzenschutzmittel.

Es ist bereits bekannt, daß bestimmte jeweils vicinal substituierte Cycloalkyl-triazolyl-alkanole, wie beispielsweise das 1-Cyclohexyl-4,4-dimethyl-5-ethoxy-2-(1,2,4-triazol-1-yl)-pentan-3-ol fungizide Eigenschaften besitzen (vergl. DE-OS 30 48 267 bzw. EP 55 833 [LeA 20 762]).

Weiterhin ist bekannt, daß auch 3-Aryl-1-(1,3-dioxanyl)-2-triazolyl-propanone und -propanole wie beispielsweise das 3-(4-Chlorphenyl)-1-(5-methyl-1,3-dioxan-5-yl)-2-(1,2,4-triazol-1-yl)-propan-1-ol fungizide Eigenschaften besitzen (vergl. DE-OS 31 13 628).
Die Wirkung dieser Verbindungen ist jedoch, insbesondere bei niedrigen Aufwandmengen und -konzentrationen nicht immer völlig zufriedenstellend.

Es wurden neue 3-Cycloalkyl-1-(1,3-dioxan-5-yl)-2-(1,2,4-triazol-1-yl)-propan-1-one und -propan-1-ole der allgemeinen Formel (I)

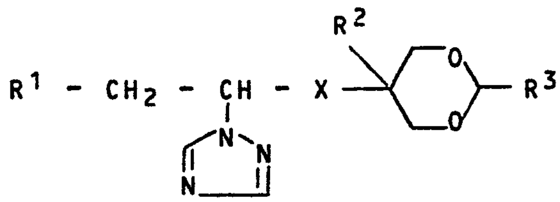

(I)

in welcher

$R^1$ für gegebenenfalls substituiertes Cycloalkyl steht,

$R^2$ für Wasserstoff oder Alkyl steht,

$R^3$ für Wasserstoff oder Alkyl steht und

X für die $-\underset{\underset{O}{\|}}{C}-$ oder die $-\underset{\underset{OH}{|}}{CH}-$Gruppe steht,

sowie deren pflanzenverträgliche Säureadditionssalze und Metallsalzkomplexe gefunden.

Die Verbindungen der Formel (I) können als diastereomere und/oder optische Isomere oder Isomerengemische unterschiedlicher Zusammensetzung anfallen. Sowohl die reinen Isomeren als auch die Isomerengemische werden erfindungsgemäß beansprucht.

Weiterhin wurde gefunden, daß man die neuen 3-Cycloalkyl-1-(1,3-dioxan-5-yl)-2-(1,2,4-triazol-1-yl)-propan-1-one und -propan-1-ole der Formel (I) erhält, wenn man entweder

a) 3-Cycloalkyl-1-(1,3-dioxan-5-yl)-2-halogen-propan-1-one der allgemeinen Formel (II),

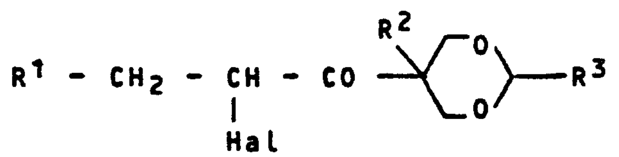

(II)

in welcher

$R^1$, $R^2$ und $R^3$ die oben angegebene Bedeutung haben und

Hal für Halogen steht

mit Triazol gegebenenfalls in Gegenwart eines Verdünnungsmittels und gegebenenfalls in Gegenwart eines Säurebindemittels umsetzt oder wenn man

b) 1-(1,3-Dioxan-5-yl)-2-(1,2,4-triazol-1-yl)-ethan-1-one der Formel (III),

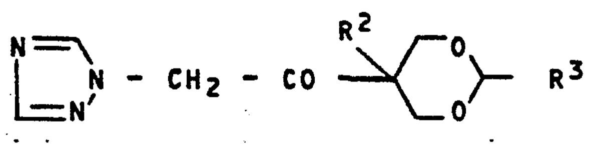

(III)

in welcher

$R^2$ und $R^3$ die oben angegebene Bedeutung haben ,

mit Cycloalkyl-methylhalogeniden der Formel (IV),

$$R^1 - CH_2 - Hal' \qquad (IV)$$

in welcher

$R^1$ die oben angegebene Bedeutung hat und

Hal' für Halogen steht,

gegebenenfalls in Gegenwart eines Verdünnungsmittels und gegebenenfalls in Gegenwart eines Säurebindemittels umsetzt oder wenn man

c) die nach Verfahren a) oder Verfahren b) erhältlichen erfindungsgemäßen 3-Cycloalkyl-1-(1,3-dioxan-5-yl)-2-(1,2,4-triazol-1-yl)-propan-1-one der Formel (Ia)

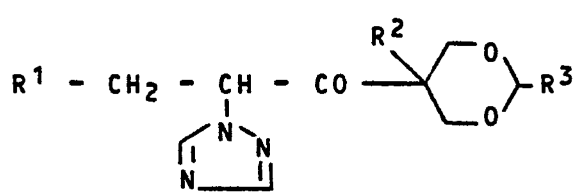

(Ia)

in welcher

$R^1$,$R^2$ und $R^3$ die oben angegebene Bedeutung haben,

nach üblichen Verfahren reduziert zu den erfindungsgemäßen 3-Cycloalkyl-1-(1,3-dioxan-5-yl)-2-(1,2,4-triazol-1-yl)-propan-1-ole der Formel (Ib),

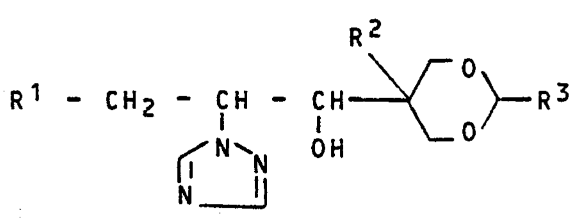

(Ib)

in welcher

$R^1$, $R^2$ und $R^3$ die oben angegebene Bedeutung haben.

An die so erhaltenen Verbindungen der allgemeinen Formel (I) kann gegebenenfalls anschließend eine Säure oder ein Metallsalz addiert werden.

Schließlich wurde gefunden, daß die neuen 3-Cycloalkyl-1-(1,3-dioxan-5-yl)-2-(1,2,4-triazol-1-yl)-propan-1-one und -propan-1-ole der allgemeinen Formel (I) fungizide Eigenschaften besitzen.
Ueberraschenderweise zeigen die erfindungsgemäßen 3-Cycloalkyl-1-(1,3-dioxan-5-yl)-2-(1,2,4-triazol-1-yl)-propan-1-one und -propan-1-ole eine bessere fungizide Wirksamkeit als die aus dem Stand der Technik bekannten Verbindungen 1-Cyclohexyl-4,4-dimethyl-5-ethoxy-2-(1,2,4-triazol-1-yl)-pentan-3-ol und 3-(4-Chlorphenyl)-1-(5-methyl-1,3-dioxan-5-yl)-2-(1,2,4-triazol-1-yl)-propan-1-ol, welches chemisch und wirkungsmäßig naheliegende Verbindungen sind.

Die erfindungsgemäßen Stoffe der Formel (I) stellen somit eine wertvolle Bereicherung des Standes der Technik dar.

Die erfindungsgemäßen 3-Cycloalkyl-1-(1,3-dioxan-5-yl)-2-(1,2,4-triazol-1-yl)-propan-1-one und -propan-1-ole sind durch die Formel (I) allgemein definiert. Bevorzugt sind Verbindungen der Formel (I), bei denen

X für die $-\overset{\overset{O}{\|}}{C}-$ oder die $-\overset{\overset{OH}{|}}{C}H$-Gruppe steht,

$R^1$ für gegebenenfalls durch geradkettiges oder verzweigtes Alkyl mit bis zu 4 Kohlenstoffatomen einfach oder mehrfach, gleich oder verschieden substituiertes Cycloalkyl mit 3 bis 9 Kohlenstoffatomen steht und

$R^2$ und $R^3$ unabhängig voneinander für Wasserstoff oder für geradkettiges oder verzweigtes Alkyl mit bis zu 4 Kohlenstoffatomen stehen.

Besonders bevorzugt sind Verbindungen der Formel (I), bei denen

X für die $-\overset{O}{\overset{\|}{C}}-$ oder die $-\overset{OH}{\overset{|}{C}}H$-Gruppe steht,

$R^1$ für jeweils gegebenenfalls ein- bis dreifach, gleich oder verschieden durch Methyl, Ethyl, n- und i-Propyl sowie n-, i-, s- und t-Butyl substituiertes Cyclopropyl, Cyclopentyl, Cyclohexyl oder Cycloheptyl steht und

$R^2$ und $R^3$ unabhängig voneinander für Wasserstoff, Methyl, Ethyl, i-Propyl oder t-Butyl stehen.

Im einzelnen seien außer den bei den Herstellungsbeispielen genannten Verbindungen die folgenden Verbindungen der allgemeinen Formel (I) genannt:

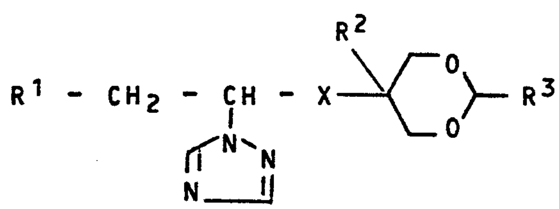
(I)

0135083

| $R^1$ | $R^2$ | $R^3$ | X |
|---|---|---|---|
| 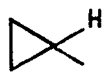 | H | H | >C=O |
| 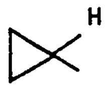 | H | H | >CH-OH |
| 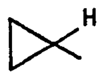 | $CH_3$ | $CH_3$ | >C=O |
| 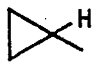 | $CH_3$ | $CH_3$ | >CH-OH |
| 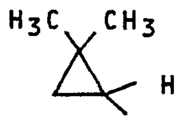 | H | H | >C=O |
| 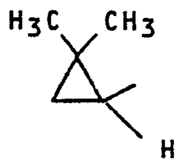 | H | H | >CH-OH |
| 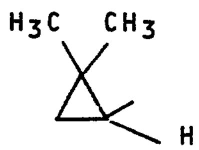 | $CH_3$ | $CH_3$ | >CH-OH |
| 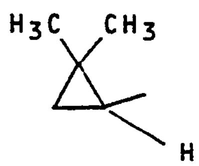 | $CH_3$ | $CH_3$ | >C=O |
| 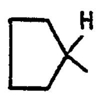 | H | H | >C=O |
| 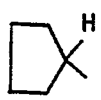 | H | H | >CH-OH |
| 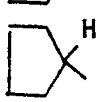 | $CH_3$ | H | >C=O |
| 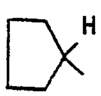 | $CH_3$ | H | >CH-OH |
| 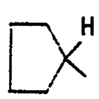 | H | $CH_3$ | >C=O |
| 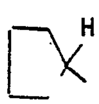 | H | $CH_3$ | >CH-OH |

| $R^1$ | $R^2$ | $R^3$ | X |
|---|---|---|---|
| 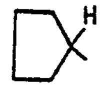 | $CH_3$ | $CH_3$ | $>C=O$ |
| 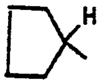 | $CH_3$ | $CH_3$ | $>CH-OH$ |
| 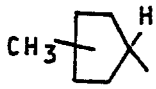 | H | H | $>CH-OH$ |
| 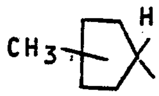 | H | H | $>C=O$ |
| 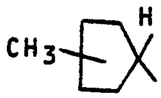 | $CH_3$ | $CH_3$ | $>C=O$ |
| 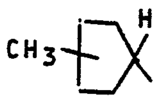 | $CH_3$ | $CH_3$ | $>CH-OH$ |
| 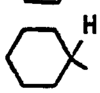 | $CH_3$ | $CH_3$ | $>C=O$ |
| 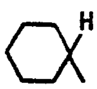 | $CH_3$ | $CH_3$ | $>CH-OH$ |
| 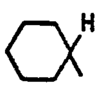 | H | H | $>C=O$ |
| 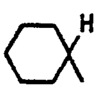 | H | H | $>CH-OH$ |
| 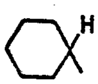 | H | $CH_3$ | $>C=O$ |
| 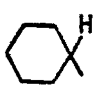 | H | $CH_3$ | $>CH-OH$ |
| 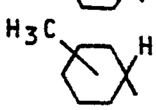 | H | H | $>C=O$ |
| 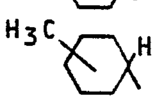 | H | H | $>CH-OH$ |
| 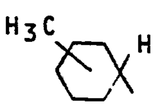 | $CH_3$ | $CH_3$ | $>C=O$ |
| 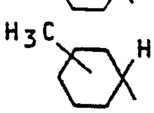 | $CH_3$ | $CH_3$ | $>CH-OH$ |

| $R^1$ | $R^2$ | $R^3$ | X |
|---|---|---|---|
| 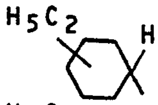 | $CH_3$ | $CH_3$ | >C=O |
| 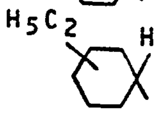 | $CH_3$ | H | >C=O |
| 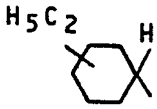 | H | $CH_3$ | >CH-OH |
| 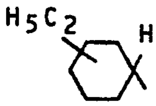 | H | $CH_3$ | >CH-OH |
| 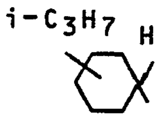 | $CH_3$ | $CH_3$ | >C=O |
| 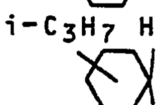 | H | $CH_3$ | >CH-OH |
| 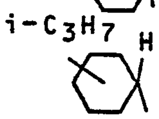 | $CH_3$ | H | >C=O |
| 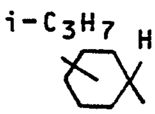 | $CH_3$ | $CH_3$ | >CH-OH |
| 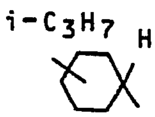 | H | H | >C=O |
| 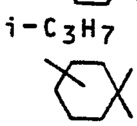 | $CH_3$ | H | >CH-OH |
| 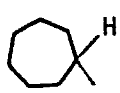 | H | H | >C=O |
| 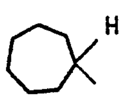 | H | H | >CH-OH |
| 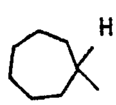 | $CH_3$ | H | >C=O |
| 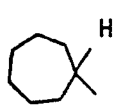 | $CH_3$ | H | >CH-OH |
| 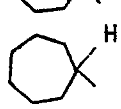 | $CH_3$ | $CH_3$ | >C=O |

| $R^1$ | $R^2$ | $R^3$ | X |
|---|---|---|---|
| 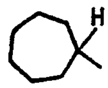 | $CH_3$ | $CH_3$ | >CH-OH |
| 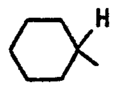 | $CH_3$ | $C(CH_3)_3$ | >CH-OH |
| 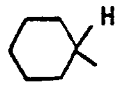 | $CH_3$ | 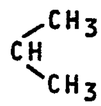 | >CH-OH |
| 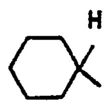 | $CH_3$ | $C_2H_5$ | >CH-OH |

Verwendet man beispielsweise 2-Brom-3-cyclohexyl-1-(1,3-dioxan-5-yl)-propan-1-on und Triazol als Ausgangsstoffe, so läßt sich der Reaktionsablauf des erfindungsgemäßen Verfahrens a) durch das folgende Formelschema darstellen:

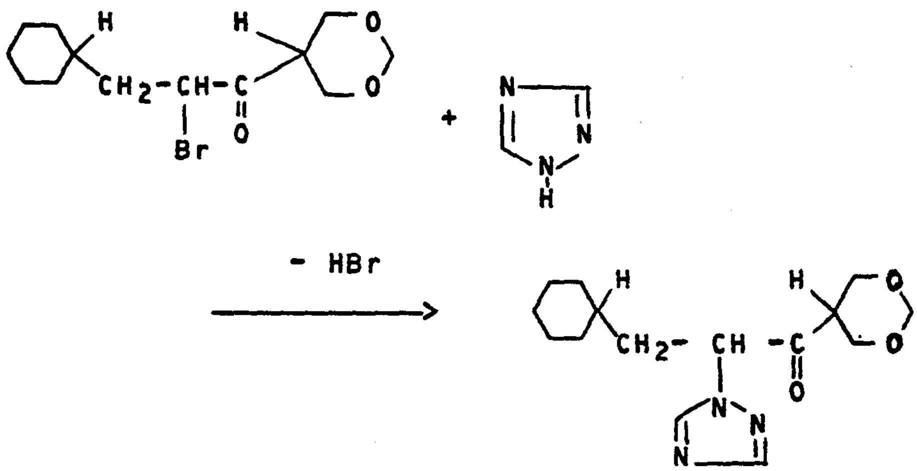


Verwendet man beispielsweise 1-(5-Methyl-1,3-dioxan-5-yl)-2-(1,2,4-triazol-1-yl)-ethan-1-on und Cyclohexylmethylbromid als Ausgangsstoffe, so läßt sich der Reaktionsablauf des erfindungsgemäßen Verfahrens b) durch das folgende Formelschema darstellen:

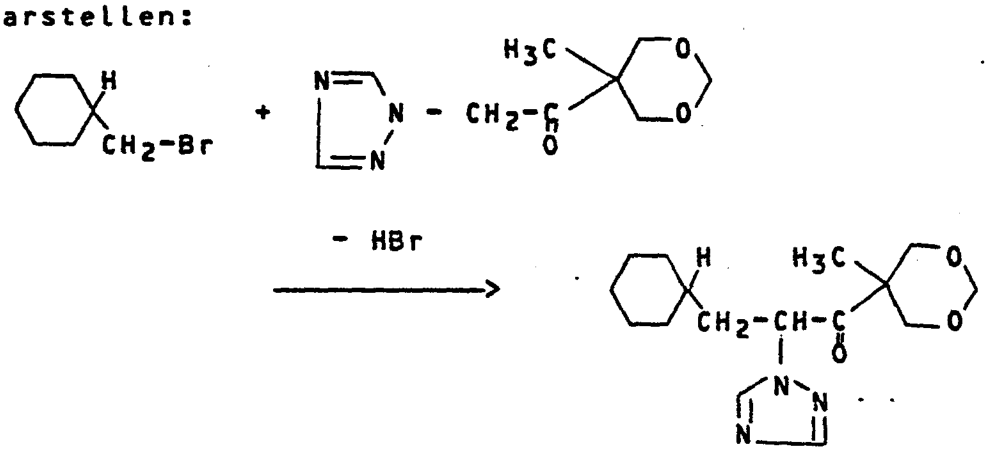

Verwendet man beispielsweise 3-Cyclohexyl-1-(2,5-dimethyl-1,3-dioxan-5-yl)-2-(1,2,4-triazol-1-yl)-propan-1-on als Ausgangsstoff und Natriumborhydrid als Reduktionsmittel, so läßt sich der Reaktionsablauf des erfindungsgemäßen Verfahrens c) durch das folgende Formelschma darstellen:

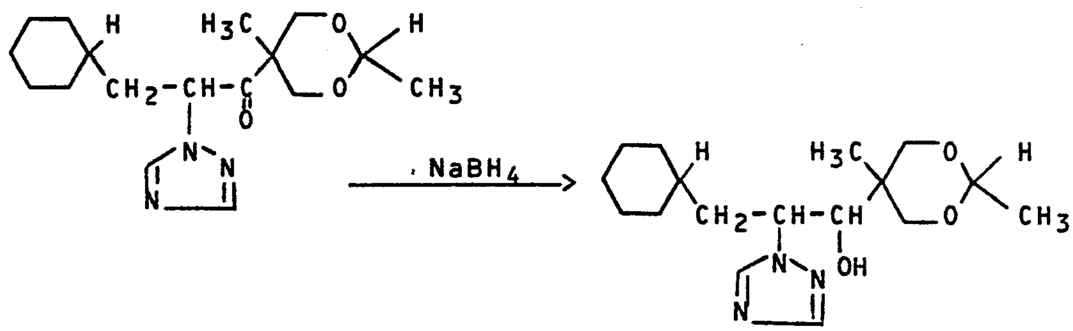


Die zur Durchführung des erfindungsgemäßen Verfahrens a) als Ausgangsstoffe benötigten 3-Cycloalkyl-1-(1,3-dioxan-5-yl)-2-halogenketone sind durch die Formel (II) allgemein definiert. In dieser Formel (II) besitzen $R^1$, $R^2$ und $R^3$ vorzugsweise diejenigen Bedeutungen, die schon bei der Beschreibung der erfindungsgemäßen Stoffe der Formel (I) als bevorzugt für diese Reste angegeben wurden. Hal steht vorzugsweise für Chlor oder Brom.

Die 3-Cycloalkyl-1-(1,3-dioxan-5-yl)-2-halogenketone der Formel (II) sind noch nicht bekannt. Man erhält sie jedoch in prinzipiell bekannter Weise, wenn man (1,3-Dioxan-5-yl)-methyl-ketone der Formel (V),

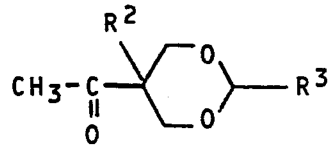 (V)

in welcher

$R^2$ und $R^3$ die oben angegebene Bedeutung haben,

zunächst in einer 1.Stufe mit Cycloalkylmethylhalogeniden der Formel (IV),

$$R^1 - CH_2 - Hal' \qquad (IV)$$

in welcher

$R^1$ die oben angegebene Bedeutung hat und

Hal' vorzugsweise für Chlor oder Brom steht

gegebenenfalls in Gegenwart eines Verdünnungsmittels wie beispielsweise Dimethylformamid und in Gegenwart einer Base wie beispielsweise Natriumhydrid bei Temperaturen zwischen 0 °C und 120°C alkyliert zu den 3-Cycloalkyl-1-(1,3-dioxan-5-yl)-propan-1-onen der Formel (VI),

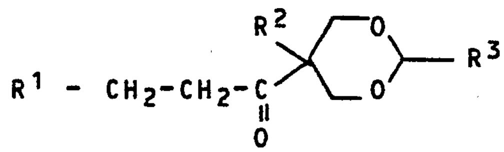
(VI)

in welcher

$R^1$,$R^2$ und $R^3$ die oben angegebene Bedeutung haben,

und diese in einer 2.Stufe mit Hilfe prinzipiell bekannter Verfahren,z.B. mit Brom in Formamid (vergl.Chem. Ber. 93, 2083 [1960]) oder mit Dioxan-Dibromid (vergl.Austr. J.Chem. 26, 1327 [1973])oder mit dem Komplex $Br_2$ x HBr x $(Pyrrolidon)_3$ (vergl.Can.J.Chem. 47, 706 [1969]) halogeniert.

(1,3-Dioxan-5-yl)-methyl-ketone der Formel (V) sind bekannt (vergl.EP-OS 44 407 und DE-OS 31 13 628).

Die zur Durchführung des erfindungsgemäßen Verfahrens b) als Ausgangsstoffe benötigten 1-(1,3-Dioxan-5-yl)-2-(1,2,4-triazol-1-yl)-ethan-1-one sind durch die Formel (III) allgemein definiert. In dieser Formel (III) besitzen $R^2$ und $R^3$ vorzugsweise diejenigen Bedeutungen, die schon bei der Beschreibung der erfindungsgemäßen Stoffe der Formel (I) als bevorzugt für dieser Reste angegeben wurden.

Die 1-(1,3-Dioxan-5-yl)-2-(1,2,4-triazol-1-yl)-ethan-1-one der Formel (III) sind ebenfalls bekannt (vergl. EP 44 407 und DE-OS 31 13 628).

Die weiterhin zur Durchführung des erfindungsgemäßen Verfahrens b) und zur Herstellung der Ausgangsstoffe der Formel (II) benötigten Cycloalkylmethylhalogenide sind durch die Formel (IV) allgemein definiert. In dieser Formel (IV) besitzt $R^1$ vorzugsweise diejenigen Bedeutungen, die schon bei der Beschreibung der erfindungsgemäßen Stoffe der Formel (I) als bevorzugt für diesen Rest angegeben wurden. Hal' steht vorzugsweise für Chlor oder Brom.

Die Cycloalkylmethylhalogenide sind allgemein bekannte Verbindungen der organischen Chemie.

Die zur Durchführung des erfindungsgemäßen Verfahrens c) als Ausgangsstoffe benötigten 3-Cycloalkyl-1-(1,3-dioxan-5-yl)-2-(1,2,4-triazol-1-yl)-propan-1-one sind durch die Formel (Ia) allgemein definiert. Die 3-Cycloalkyl-1-(1,3-dioxan-5-yl)-2-(1,2,4-triazol-1-yl)-propan-1-one der Formel (Ia) sind erfindungsgemäße Verbindungen und erhältlich nach den erfindungsgemäßen Verfahren a) und b).

Als Verdünnungsmittel kommen für das erfindungsgemäße Verfahren a) inerte organische Lösungsmittel infrage. Hierzu gehören vorzugsweise chlorierte Kohlenwasserstoffe, wie

z.B. Dichlormethan, Chloroform, Tetrachlorkohlenstoff oder Chlorbenzol, Amide, wie Dimethylformamid, Dimethylacetamid oder N-Methylformanilid, Nitrile, wie Acetonitril oder Propionitril, sowie die hochpolaren Lösungsmittel Dimethylsulfoxid, Sulfolan oder Hexamethylphosphorsäuretriamid.

Das erfindungsgemäße Verfahren a) kann in Gegenwart eines Säurebindemittels vorgenommen werden. Man kann alle üblicherweise verwendbaren anorganischen oder organischen Säurebinder zugeben, wie Alkalicarbonate, z.B. Natriumcarbonat und Kaliumcarbonat, wie niedere tertiäre Alkylamine, Cycloalkylamine oder Aralkylamine, z.B. Triethylamin, N,N-Dimethylcyclohexylamin und N,N-Dimethylbenzylamin. Ebenfalls möglich ist ein entsprechender Ueberschuß an der Triazol- Komponente.

Die Reaktionstemperaturen können bei dem erfindungsgemäßen Verfahren a) in einem größeren Bereich variiert werden. Im allgemeinen arbeitet man zwischen 0°C und 150°C, vorzugsweise zwischen 35 und 90°C.

Zur Durchführung des erfindungsgemäßen Verfahrens a) setzt man auf 1 Mol der Verbindungen der allgemeinen Formel (II) vorzugsweise 1 bis 3 Mol 1,2,4-Triazol ein. Die Isolierung der Verbindungen der allgemeinen Formel (Ia) erfolgt nach üblichen Methoden.

Als Verdünnungsmittel für das erfindungsgemäße Verfahren b) kommen ebenfalls inerte organische Lösungsmittel infrage. Hierzu gehören vorzugsweise aromatische Kohlenwasserstoffe, wie Benzol, Toluol oder Xylol, halogenierte Kohlenwasserstoffe, wie Methylenchlorid, Chloroform, Tetrachlorkohlenstoff oder Chlorbenzol, Ester, wie Essigester, Amide, wie Dimethylformamid sowie Dimethylsulfoxid.

Das erfindungsgemäße Verfahren b) wird üblicherweise in Gegenwart einer Base durchgeführt. Hierbei können alle üblichen organischen und insbesondere anorganischen Basen eingesetzt werden, beispielsweise Alkalihydroxide oder Alkalicarbonate, wie Natriumhydroxid, Kaliumhydroxid oder Kaliumcarbonat.

Die Reaktionstemperaturen können bei der Durchführung des erfindungsgemäßen Verfahrens b) in einem größeren Bereich variiert werden. Im allgemeinen arbeitet man zwischen 0°C und 120°C, vorzugsweise zwischen 20°C und 100°C.

Zur Durchführung des erfindungsgemäßen Verfahrens b) setzt man auf 1 Mol der Verbindungen der allgemeinen Formel (III) vorzugsweise äquimolare Mengen an Cycloalkylmethylhalogenid der Formel (IV) und äquimolare Mengen an Säurebindemittel ein. Die Aufarbeitung und Isolierung der Verbindungen der allgemeinen Formel (Ia) erfolgt nach üblichen Methoden.

Die Durchführung des erfindunsgemäßen Verfahrens c) (Reduktion der Keto-Gruppe) erfolgt in üblicher Weise, wie z.B. durch Umsetzung der Verbindungen der Formel (Ia) mit komplexen Hydriden, gegebenenfalls in Gegenwart eines Verdünnungsmittels; oder durch Umsetzung mit Aluminiumisopropylat in Gegenwart eines Verdünnungsmittels; oder durch Umsetzung mit Wasserstoff in Gegenwart eines Katalysators und gegebenenfalls in Gegenwart eines Verdünnungsmittels.

Arbeitet man mit komplexen Hydriden, so kommen als Verdünnungsmittel für die Reduktions-Reaktion polare organische Lösungsmittel infrage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30°C , vorzugsweise bei 0 bis 20°C durchgeführt. Hierzu setzt man auf 1 Mol

des Ketones der Formel (Ia) etwa 1 Mol eines komplexen Hydrids, wie Natriumborhydrid oder Lithiumalanat, ein. Zur Isolierung der reduzierten Verbindungen der Formel (Ib) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die oben erwähnte Reduktion bevorzugt Alkohole, wie Isopropanol, oder inerte Kohlenwasserstoffe, wie Benzol, infrage. Die Reaktionstemperaturen können in einem größeren Bereich variiert werden; im allgemeinen arbeitet man zwischen 20 und 120°C, vorzugsweise zwischen 50 bis 100°C. Zur Durchführung der Reaktion setzt man auf 1 Mol des Ketons der Formel (Ia) etwa 1 bis 2 Mol Aluminiumisopropylat ein. Zur Isolierung der reduzierten Verbindungen der Formel (Ib) wird das überschüssige Lösungsmittel durch Destillation im Vakuum entfernt und die entstandene Aluminiumverbindung mit verdünnter Schwefelsäure oder Natronlauge zersetzt. Die weitere Aufarbeitung erfolgt in üblicher Weise.

Arbeitet man mit Wasserstoff, so kommen als Verdünnungsmittel für die durchzuführende Reduktion polare organische Lösungsmittel infrage. Hierzu gehören vorzugsweise Alkohole, wie Methanol und Ethanol; sowie Nitrile, wie Acetonitril. Die Umsetzung wird in Gegenwart eines Katalysators vorgenommen. Vorzugsweise werden Edelmetall-, Edelmetalloxid- bzw. Edelmetallhydroxid-Katalysatoren oder sogenannte 'Raney-Katalysatoren' verwendet, insbesondere Platin, Platinoxid und Nickel. Die Reaktionstemperaturen können in einem größeren Bereich variiert werden. Im allgemeinen arbeitet man zwischen 20 und 50°C. Die Reaktion kann bei Normaldruck, aber auch bei erhöhtem Druck, beispielsweise 1 bis 2 atü, durchgeführt werden. Zur Durchfürhrung der Reaktion setzt man auf 1 Mol der Verbindung der Formel (Ia) etwa 1 Mol Wasserstoff und 0,1 Mol Katalysator ein. Zur Isolierung der reduzierten Verbindungen der Formel (Ib) wird vom Katalysator abfiltriert und das Lösungsmittel im Vakuum entfernt. Die weitere Aufarbeitung erfolgt in üblicher Weise.

Die erfindunsggemäß herstellbaren Verbindungen der Formel (I) können in Säureadditionssalze bzw. Metallsalz-Komplexe überführt werden.

Zur Herstellung von pflanzenverträglichen Säureadditionssalzen der Verbindungen der allgemeinen Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, sowie Sulfonsäuren, wie z.B. p-Toluolsulfonsäure und 1,5-Naphthalindisulfonsäure.

Die Säureadditionssalze der Verbindungen der allgemeinen Formel (I) können in einfacher Weise nach üblichen Salzbildungsmethoden, z.B. durch Lösen einer Verbindung der allgemeinen Formel (I) in einem geeigneten organischen Lösungsmittel und Hinzufügen der Säure, z.B. Chlorwasserstoffsäure, erhalten werden und in bekannter Weise, durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. Chlorwasserstoffsäure und Bromwasserstoffsäure, ferner Phosphorsäure, Salpetersäure und Schwefelsäure.

Die Metallsalz-komplexe von Verbindungen der allgemeinen Formel (I) können in einfacher Weise nach üblichen Verfahren erhalten werden, so z.B. durch Lösen des Metallsalzes in Alkohol, wie z.B. Ethanol und Hinzufügen zur Verbindung der allgemeinen Formel (I). Man kann die Metallsalz-komplexe in bekannter Weise, z.B. durch Abfiltrieren , isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten , wie zum Beispiel gegen den Erreger der Blattfleckenkrankheit der Gerste (Pyrenophora teres), von Obstkrankheiten, wie zum Beispiel gegen den Erreger des Apfelmehltaus (Podosphaera leucotricha) oder von Gemüsekrankheiten, wie zum Beispiel gegen den Erreger des Gurkenmehltaus (Sphaerotheca fuliginea) eingesetzt werden.
Daneben besitzen die erfindungsgemäßen Wirkstoffe auch eine gute Wirksamkeit gegen Getreidemehltauarten und gegen Erreger von Reiskrankheiten, wie z.B. Pyricularia oryzae.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele:

Beispiel 1 :

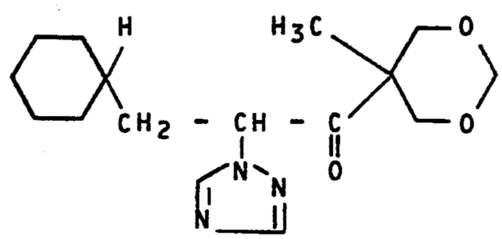

(Verfahren b)

Zu einer Lösung von 11,0g (0,05 Mol) 1-(5-Methyl-1,3-dioxan-5-yl)-2-(1,2,4-triazol-1-yl)-ethan-1-on in 40 ml Dimethylsulfoxid gibt man unter Rühren 2,96g (0,05 Mol) gepulvertes Kaliumhydroxid und 3 ml Wasser. Nach 10 Minuten gibt man 9,6g (0,05 Mol) Brommethylcyclohexan (90 %-ig) dazu und rührt 6 Stunden bei 50°C. Zur Aufarbeitung gießt man das erkaltete Reaktionsgemisch auf 500 ml Wasser, extrahiert 2 mal mit je 200 ml Diethylether, wäscht die vereinigten Etherextrakte zweimal mit je 100 ml Wasser, trocknet über Natriumsulfat und entfernt das Lösungsmittel im Vakuum. Der ölige Rückstand kristallisiert aus Dichlormethan/Diethylether. Man erhält 8,0g (52,1g der Theorie) an 3-Cyclohexyl-1-(5-methyl-1,3-dioxan-5-yl)-2-(1,2,4-triazol-1-yl)-propan-1-on vom Schmelzpunkt 68 - 71 °C.

Beispiel 2:

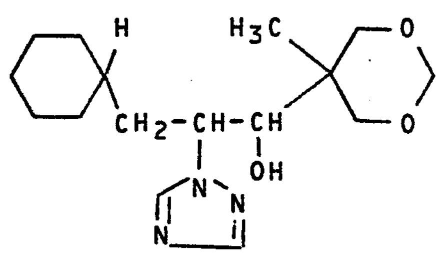

(Verfahren c)

Zu einer Mischung von 6,0g (0,02 Mol) 3-Cyclohexyl-1-(5-methyl-1,3-dioxan-5-yl)-2-(1,2,4-triazol-1-yl)-propan-1-on, 2,3g gepulvertem Calciumchlorid und 30 ml Ethanol gibt man unter Eiskühlung eine Lösung von 0,7g (0,02 Mol) Natriumborhydrid in 3 ml 0,1 normaler wässriger Natronlauge. Nach beendeter Gasentwicklung löst man den entstandenen Niederschlag durch Zugabe von 30 ml Ethanol und 50 ml verdünnter Salzsäure wieder auf, engt die Lösung im Vakuum auf 60 ml ein, neutralisiert mit wässriger Bicarbonatlösung und extrahiert zweimal mit je 100 ml Methylenchlorid. Die vereinigten organischen Phasen werden mit Wasser gewaschen, über Natriumsulfat getrocknet und im Vakuum vom Lösungsmittel befreit. Man erhält 6,3g (100 % der Theorie ) an 3-Cyclohexyl-1-(5-methyl-1,3-dioxan-5-yl)-2-(1,2,4-triazol-1-yl)-propan-1-ol als farbloses Oel.

IR($cm^{-1}$): 3667,2999,2853,2772,1670,1612,1510,1480,
1450,1384,1307,1277,1236,1164,1139,1087,
1070,1029, 990, 932, 922, 888, 854, 725, 675

Anwendungsbeispiele:

In den nachsteneden Anwendungsbeispielen werden die folgenden Verbindungen als Vergleichssubstanzen eingesetzt:

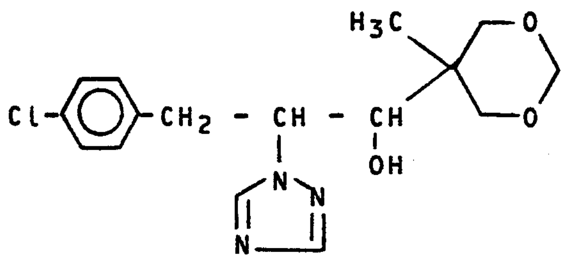
(A)

3-(4-Chlorphenyl)-1-(5-methyl-1,3-dioxan-5-yl)-2-(1,2,4-triazol-1-yl)-propan-1-ol

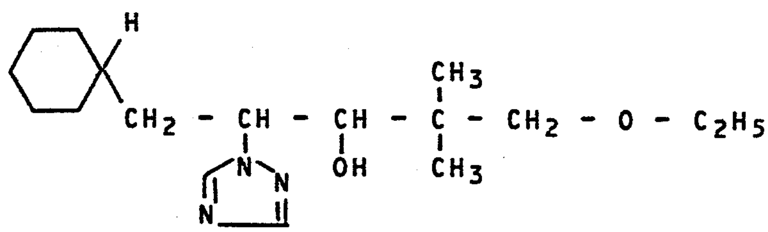
(B)

1-Cyclohexyl-4,4-dimethyl-5-ethoxy-2-(1,2,4-triazol-1-yl)-pentan-3-ol

Beispiel A

Sphaerotheca-Test (Gurke) / protektiv /

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigt in diesem Test z.B. die Verbindung gemäß folgendem Herstellungsbeispiel: 2.

Beispiel B

Podosphaera-Test (Apfel) / protektiv /

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23°C und einer relativen Luftfeuchtigkeit von ca. 70 % aufgestellt.

9 Tage nach der Inokulation erfolgt die Auswertung.

Eine deutliche Überlegenheit in der Wirksamkeit gegenüber dem Stand der Technik zeigt in diesem Test z.B. die Verbindung gemäß folgendem Herstellungsbeispiel: 2.

Beispiel C

Pyrenophora teres-Test (Gerste) / protektiv /

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigt in diesem Test z.B. die Verbindung gemäß folgendem Herstellungsbeispiel: 2.

Patentansprüche

1. 3-Cycloalkyl-1-(1,3-dioxan-5-yl)-2-(1,2,4-triazol-1-yl)-propan-1-one und -propan-1-ole der allgemeinen Formel (I)

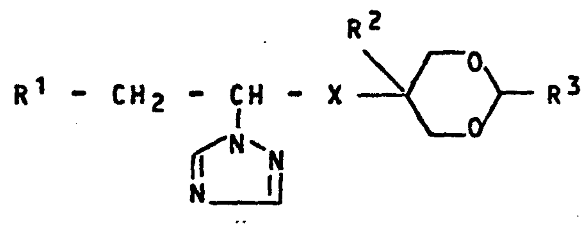 (I)

in welcher

$R^1$ für gegebenenfalls substituiertes Cycloalkyl steht,

$R^2$ für Wasserstoff oder Alkyl steht,

$R^3$ für Wasserstoff oder Alkyl steht und

X für die $-\underset{\underset{O}{\|}}{C}-$ oder die $-\underset{\underset{OH}{|}}{C}H$-Gruppe steht,

sowie deren pflanzenverträgliche Säureadditionssalze und Metallsalzkomplexe.

2. Verbindungen der Formel (I) in Anspruch 1, wobei

$R^1$ für gegebenenfalls durch geradkettiges oder verzweigtes Alkyl mit bis zu 4 Kohlenstoffatomen einfa oder mehrfach, gleich oder verschieden substituiert Cycloalkyl mit 3 bis 9 Kohlenstoffatomen steht,

$R^2$ und $R^3$ unabhängig voneinander für Wasserstoff oder für geradkettiges oder verzweigtes Alkyl mit bis zu 4 Kohlenstoffatomen stehen, und

X für die $-\overset{\overset{O}{\|}}{C}-$ oder die $-\overset{\overset{OH}{|}}{C}H$-Gruppe steht.

3. Verbindungen der Formel (I) in Anspruch 1, wobei

$R^1$ für jeweils gegebenenfalls ein- bis dreifach, gleic oder verschieden durch Methyl, Ethyl, n- und i-Propyl sowie n-, i-, s- und t-Butyl substituiertes Cyclopropyl, Cyclopentyl, Cyclohexyl oder Cyclohept steht,

$R^2$ und $R^3$ unabhängig voneinander für Wasserstoff, Methyl, Ethyl, i-Propyl oder t-Butyl stehen, und

X für die -CO- oder -CH(OH)-Gruppe steht.

4. Verfahren zur Herstellung von 3-Cycloalkyl-1-(1,3-dioxan-5-yl)-2-(1,2,4-triazol-1-yl)-propan-1-onen und -propan-1-olen der allgemeinen Formel (I)

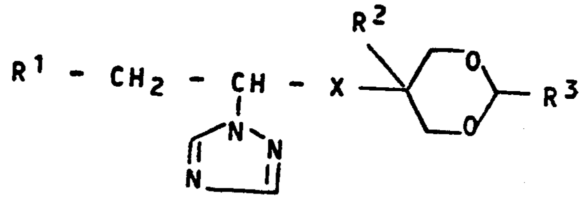

(I)

in welcher

$R^1$ für gegebenenfalls substituiertes Cycloalkyl steht,

$R^2$ für Wasserstoff oder Alkyl steht,

$R^3$ für Wasserstoff oder Alkyl steht und

X für die $-\underset{\displaystyle O}{\underset{\|}{C}}-$ oder die $-\underset{\displaystyle OH}{\underset{|}{CH}}-$Gruppe steht,

dadurch gekennzeichnet, daß man entweder

a) 3-Cycloalkyl-1-(1,3-dioxan-5-yl)-2-halogen-keton der allgemeinen Formel (II),

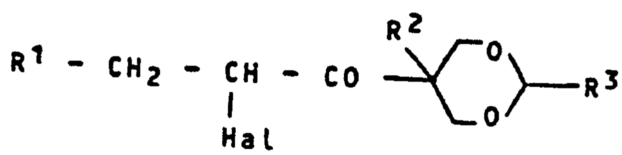

(II)

in welcher

$R^1$,$R^2$ und $R^3$ die oben angegebene Bedeutung haben und

Hal für Halogen steht

mit Triazol gegebenenfalls in Gegenwart eines Verdünnungsmittels und gegebenenfalls in Gegenwart eines Säurebindemittels umsetzt oder wenn man

b) 1-(1,3-Dioxan-5-yl)-2-(1,2,4-triazol-1-yl)-ethan-1-one der Formel (III),

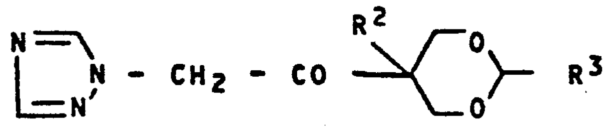

(III)

in welcher

$R^2$ und $R^3$ die oben angegebene Bedeutung haben ,

mit Cycloalkyl-methylhalogeniden der Formel (IV),

$$R^1 - CH_2 - Hal' \qquad (IV)$$

in welcher

$R^1$ die oben angegebene Bedeutung hat und

Hal' für Halogen steht,

gegebenenfalls in Gegenwart eines Verdünnungsmittels und gegebenenfalls in Gegenwart eines Säurebindemittels umsetzt oder wenn man

c) die nach Verfahren a) oder Verfahren b) erhältlichen erfindungsgemäßen 3-Cycloalkyl-1-(1,3-dioxan-5-yl)-2-(1,2,4-triazol-1-yl)-propan-1-one der Formel (Ia)

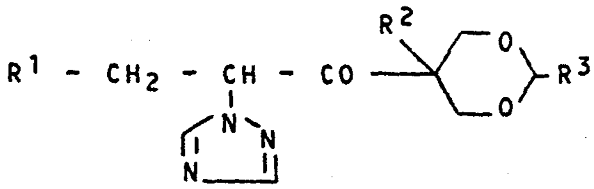
(Ia)

in welcher

$R^1$,$R^2$ und $R^3$ die oben angegebene Bedeutung haben,

nach üblichen Verfahren reduziert zu den erfindungsgemäßen 3-Cycloalkyl-1-(1,3-dioxan-5-yl)-2-(1,2,4-triazol-1-yl)-propan-1-ole der Formel (Ib),

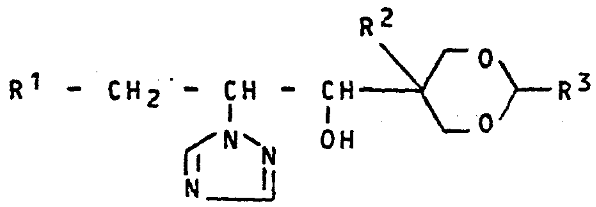
(Ib)

in welcher

$R^1$,$R^2$ und $R^3$ die oben angegebene Bedeutung haben,

und gegebenenfalls noch an die so erhaltenen Verbindungen der allgemeinen Formel (I) anschließend eine Säure oder ein Metallsalz addiert.

5. Fungizide Mittel, gekennzeichnet durch einen Gehalt an mindestens einem 3-Cycloalkyl-1-(1,3-dioxan-1-yl)-2-1,2,4-triazol-1-yl)-propan-1-on oder -propan-1-ol der Formel (I) in Ansprüchen 1 und 4.

6. Verfahren zur Bekämpfung von Pilzen, dadurch gekennzeichnet, daß man 3-Cycloalkyl-1-(1,3-dioxan-5-yl)-2-(1,2,4-triazol-1-yl)-propan-1-one oder -propan-1-ole der Formel (I) in Ansprüchen 1 und 4 auf Pilze oder ihren Lebensraum einwirken läßt.

7. Verwendung von 3-Cycloalkyl-(1,3-dioxan-5-yl)-2-(1,2,4-triazol-1-yl)-propan-1-onen oder -propan-1-olen der Formel (I) in Ansprüchen 1 und 4 als Pflanzenschutzmittel.

8. Verwendung von 3-Cycloalkyl-1-(1,3-dioxan-5-yl)-2-(1,2,4-triazol-1-)-propan-1-onen oder -propan-1-olen der Formel (I) in Ansprüchen 1 und 4 zur Bekämpfung von Pilzen.

9. Verfahren zur Herstellung von fungiziden Mitteln, dadurch gekennzeichnet, daß man 3-Cycloalkyl-1-(1,3-dioxan-5-yl)-2-(1,2,4-triazol-1-yl)-propan-1-one oder propan-1-ole der Formel (I) in Ansprüchen 1 und 4 mit Streckmitteln und/oder oberflächenaktiven Mitteln vermischt.